# EUROPEAN PATENT SPECIFICATION

(11) **EP 1 132 391 B1**
(45) Date of publication and mention of the grant of the patent: **15.11.2006**
(21) Application number: 00956989.8
(22) Date of filing: 07.09.2000
(51) Int. Cl.: C07D 501/04, C07D 501/59, C07D 501/00, C07D 501/22

(54) **PROCESS FOR THE PREPARATION OF 3-SULFONYLOXY-3-CEPHEM COMPOUNDS**
HERSTELLUNGSVERFAHREN FÜR 3-SULFONYLOXY-3-CEPHEM VERBINDUNGEN
PROCEDE DE PREPARATION DE COMPOSES 3-SULFONYLOXY-3-CEPHEM

(30) Priority: 20.09.1999 JP 26615899
(43) Date of publication of application: 12.09.2001
(73) Proprietor: OTSUKA KAGAKU KABUSHIKI KAISHA, Osaka-shi, Osaka-fu 540-0021 (JP)
(72) Inventor: KAMEYAMA, Yutaka, Otsuka Kagaku Kabushiki Kaisha, Tokuima-shi, Tokushima 771-0193 (JP)
(74) Representative: Barz, Peter
(86) International application number: PCT/JP2000/006079
(87) International publication number: WO 2001/021622

(56) References cited:
- JP-A- 7 061 987
- JP-A- 60 084 292
- US-A- 4 713 378
- YOSHIDA Y ET AL: "Synthesis and anti-Helicobacter Pylori Activity of FR182024, a New Cephem Derivative" BIOORGANIC & MEDICINAL CHEMISTRY LETTERS, OXFORD, GB, vol. 9, no. 21, 1 November 1999 (1999-11-01), pages 3123-3126, XP004181019 ISSN: 0960-894X
- YOSHIKI YOSHIDA, KEIJI MATSUDA, HIROSHI SASAKI, YOSHIMI MATSUMOTO, SATORU MATSUMOTO, SHUICHI TAWARA, HISASHI TAKASUGI: "Studies on Anti-Helicobacter pylori Agents. Part 2: New cephem Derivatives" BIOORGANIC AND MEDICINAL CHEMISTRY, vol. 8, 2000, pages 2317-2335, XP001057079
- R. SCARTAZZINI, P. SCHNEIDER, H. BICKEL: "Neue b-Lactam-Antibiotica. Über die Funktionalisierung der Cephem-3-Stellung mittels Schwefel oder Stickstoff " HELVETICA CHIMICA ACTA, vol. 58, 1975, pages 2437-2450, XP001056840
- LEE, KANG, KIM, CHA, SHIN, CHO, KIM, HONG: "Preparation of Ceph-3-em Esters unaccompanied by D3 to D2 Isomerization of the Cephalosporin Derivatives" SYNTHETIC COMMUNICATIONS, vol. 29, no. 11, 15 April 1999 (1999-04-15), pages 1873-1887, XP001057352
- PATENT ABSTRACTS OF JAPAN vol. 003, no. 118 (C-060), 4 October 1979 (1979-10-04) & JP 54 100392 A (SANKYO CO LTD), 8 August 1979 (1979-08-08)
- VITTORIO FARINA ET AL.: 'A general route to 3-functionalized 3-norcephalosporins' J. ORG. CHEM. vol. 54, 1989, pages 4962 - 4966, XP002935180

## Description

### TECHNICAL FIELD

The present invention relates to a method of producing a 3-sulfonyloxy-3-cephem compound shown by the formula (3).

A 3-sulfonyloxy-3-cephem compound is an important intermediate for synthesizing useful antibacterial agents which have wide antibacterial spectra such as orally administrated antimicrobial agent ceftibuten which is widely used (Handbook of Latest Antibiotics, 9th ed., Katsuji Sakai, p.85, 1994).

### BACKGROUND ART

A 3-sulfonyloxy-3-cephem compound shown by the formula (3) is generally produced by reacting a 3-hydroxy-3-cephem derivative with sulfonyl halide or sulfonic acid anhydride in the presence of an organic base.

In case a sulfonyl halide shown by the formula (2) (R⁴ is methyl or trifluoromethyl) is used, however, a large amount of Δ 2-cephem compound which is an isomer concerning its double bond is often produced as a by-product as described in J. Org. Chem., 54, 4962 (1989) (See Reference Example 1).

In order to suppress the by-production of the Δ2-cephem compound, the reaction must be carried out at a very low temperature or using a special organic base such as Huenig base. Even in case R⁴ is p-tolyl, as described in Chemistry and Biology of β-Lactam Antibiotics, 164 (1982), where the reaction is carried out using pyridine which is a relatively mild aromatic base as an organic base, the product is a mixture comprising Δ3-cephem and Δ 2-cephem compounds.

In Pure & Appl. Chem., 59, 1041 (1987), the methanesulfonylation is described using dimethylformamide as a reaction solvent and using an organic base in a manner similar to the above. By the method described in the literature, however, a Δ 2-cephem compound is produced as a by-product at about 10% at 0 to 5°C; at about 4% even near -30°C (see Reference Examples 2 and 3).

Formation of the Δ2-cephem compound as a by-product leads to the decrease in purity and a yield of the Δ3-cephem compound which is the active mother nucleus of a cephalosporin, and accompanies great difficulty in the purification step. By a reaction using an organic base, all the base is dissolved in the reaction solvent *ab initio,* and the isomerization with respect to the double bond proceeds by the elimination of the hydrogen atom at C2 position by the base, so that a Δ2-cephem compound can be easily formed as a by-product. This is a big problem for the industrialization of this reaction.

An object of the present invention is to provide a method which allows more simply, practically, in high purity and a high yield producing 3-sulfonyloxy-Δ3-cephem compound avoiding the problem of the by-production of a Δ2-cephem compound.

### DISCLOSURE OF THE INVENTION

The present invention provides a method for producing a 3-sulfonyloxy-3-cephem compound shown by the formula (3) comprising reacting a 3-hydroxy-3-cephem compound shown by the formula (1) with a sulfonyl halide shown by the formula (2) in an organic solvent in the presence of an alkali metal carbonate or alkaline earth metal carbonate 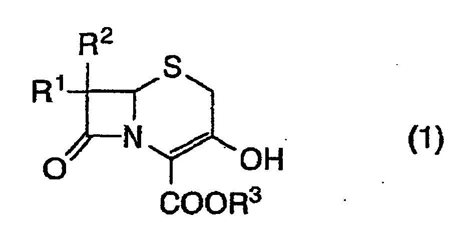 [R¹ is hydrogen atom, halogen atom, protected amino group or ArCH=N- (Ar is a substituted or unsubstituted aryl group); R² is hydrogen atom, halogen atom, C₁-C₄-alkoxy group, C₁-C₄-acyl group, protected hydroxy group or C₁-C₄-alkyl which may have protected hydroxyl group as a substituent; R³ is hydrogen atom or carboxylic acid protective group]

**R**^{**4**}**SO**_{**2**}**X** **(2)**

(R⁴ is C₁-C₄-alkyl which may have a substituent or aryl which may have a substituent; X is halogen atom) 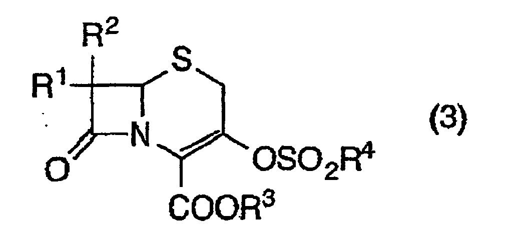 (R¹ to R⁴ are same as above).

The inventors discovered a reaction system which allows a mild reaction which allows the industrialization using an inorganic base such as alkali metal carbonate or alkaline earth metal carbonate without using an organic base which can cause isomerization with respect to the double bond for producing a 3-sulfonyloxy-3-cephem compound, and the present invention was accomplished by the above findings.

According to the present invention, a 3-sulfonyloxy-3-cephem compound shown by the formula (3) can be produced in high purity and a high yield substantially without forming a Δ2-cephem compound.

Examples of the groups described in the present specification are as follows unless particularly mentioned.

Halogen atom means fluorine, chlorine, bromine and iodine.

C₁-C₄-alkyl group means, for example, a straight-chain or branched alkyl groups having 1 to 4 carbon atoms such as methyl, ethyl, n-propyl, isopropyl, n-butyl, isobutyl, sec-butyl and tert-butyl. Aryl group means, for example, phenyl and naphthyl.

Exemplary of the protected amino represented by R¹ are amido groups such as phenoxyacetamido, p-methylphenoxyacetamido, p-methoxyphenoxyacetamido, p-chlorophenoxyacetamido, p-bromophenoxyacetamido, phenylacetamido, p-methylphenylacetamido, p-methoxyphenylacetamido, p-chlorophenylacetamido, p-bromophenylacetamido, phenylmonochloroacetamido, phenyldichloroacetamido, phenylhydroxyacetamido, phenylacetoxyacetamido, α-oxophenylacetamido, thienylacetamido, benzamido, p-methylbenzamido, p-tert-butylbenzamido, p-methoxybenzamido, p-chlorobenzamido, p-bromobenzamido, phenylglycylamido, phenylglycylamido having protected amino, p-hydroxyphenylglycylamido, p-hydroxyphenylglycylamido having protected amino and/or protected hydroxyl, etc.; imido groups such as phthalimido, nitrophthalimido, etc., in addition to the groups disclosed in Theodora W. Greene, 1981 "Protective Groups in Organic Synthesis" (hereinafter referred to merely as the "literature"), Chap. 7 (pp. 218~287). Examples of protective groups for the amino of phenylglycylamido group and p-hydroxyphenylglycylamido group are those disclosed in the literature, Chap. 7 (pp. 218~287). Examples of protective groups for the hydroxyl of p-hydroxyphenylglycylamido group are those disclosed in the literature, Chap.2 (pp. 10~72).

Examples of the C₁-C₄-alkoxyl represented by R² are straight-chain or branched alkoxyl groups having 1 to 4 carbon atoms such as methoxy, ethoxy, n-propoxy, isopropoxy, n-butoxy, isobutoxy, sec-butoxy and tert-butoxy.

Exemplary of the C₁-C₄-acyl represented by R² are straight-chain or branched acyl groups having 1 to 4 carbon atoms such as formyl, acetyl, propionyl, butyryl and isobutyryl.

Examples of protective groups for the protected hydroxyl in the C₁-C₄-alkyl represented by R² and substituted with hydroxyl or protected hydroxyl, and for the protected hydroxyl represented by R² are those disclosed in the literature, Chap. 2 (pp. 10~72). The substituted C₁-C₄-alkyl represented by R² may have as its substituent(s) one or at least two same or different groups selected from among hydroxyl and the protected hydroxyl groups. Such substituent(s) may be positioned on at least one carbon atom of the alkyl.

Exemplary of the carboxylic acid protective group represented by R³ are allyl, benzyl, p-methoxybenzyl, p-nitrobenzyl, diphenylmethyl, trichloromethyl, trichloroethyl, tert-butyl, and those disclosed in the literature, Chap. 5 (pp. 152~192) .

Exemplary of the substituent which may be substituted in the aryl represented by Ar and in the C₁-C₄-alkyl group or aryl group represented by R⁴ include a halogen atom, nitro, cyano, aryl group, C₁-C₄-alkyl group, mono-C₁-C₄-alkylamino, di- C₁-C₄-alkylamino, mercapto, alkylthio group or arylthio group shown by R⁵S- (R⁵ is lower alkyl or aryl), formyloxy, acyloxy group shown by R⁵COO- (R⁵ is as defined above), formyl, acyl group shown by R⁵CO- (R⁵ is as defined above), alkoxy group or aryloxy group shown by R⁵O- (R⁵ is as defined above), carboxyl, alkoxycarbonyl group or aryloxycarbonyl group shown by R⁵OCO- (R⁵ is as difined above), and the like. The C₁-C₄-alkyl group or aryl group in R⁴ can be substituted by substituents of the same or different kinds selected from among the above substituents, and at least one substituent may be substituted in the same or different carbon.

A 3-hydroxy-3-cephem compound used as the starting material in the present invention shown by the formula (1) can be produced, for example, by the method which is described in Chem. Lett., 1867 (1990).

According to the present invention, a 3-sulfonyloxy-3-cephem compound shown by the formula (3) can be produced by reacting a 3-hydroxy-3-cephem compound shown by the formula (1) with a sulfonyl halide shown by the formula (2) in the presence of an alkali metal carbonate or alkaline earth metal carbonate.

The alkali metal carbonate or alkaline earth metal carbonate includes sodium carbonate, potassium carbonate, lithium carbonate, magnesium carbonate, calcium carbonate, barium carbonate, strontium carbonate, and the like. Although these carbonates can be used in any form including powder and agglomerate, it is more preferable to use powder. Although a wide range of particle size can be used for the powder of an alkali metal carbonate or alkaline earth metal carbonate, it is preferable to use powder having a size of about 10 to 500 mesh. A molar ratio of the alkali metal carbonate or alkaline earth metal carbonate to the compound shown by the formula (1) is usually about 0.1 to 50, preferably about 0.5 to 10.

The sulfonyl halide shown by the formula (2) includes methanesulfonyl chloride, trifluoromethanesulfonyl chloride, p-toluenesulfonyl chloride, and the like. A molar ratio of the sulfonyl halide to the compound shown by the formula (1) is usually about 0.1 to 50, preferably about 1 to 10.

It is preferable to carry out the reaction according to the present invention in an amide solvent or cyclic ether solvent, or a mixture of these, or in a mixture of these solvent and other organic solvent(s). The amide solvent or cyclic ether solvent which can be used includes amides such as dimethylformamide (DMF), diethylformamide and dimethylacetamide (DMA), cyclic amides such as N-methylpyrrolidinone (NMP), cyclic ethers such as dioxane and dioxolane.

As the organic solvents conjointly usable, there are, for example, C₁-C₄-alkyl esters of C₁-C₄-carboxylic acids such as methyl formate, ethyl formate, propyl formate, butyl formate, methyl acetate, ethyl acetate, propyl acetate, butyl acetate, methyl propionate and ethyl propionate; ketones such as acetone, methyl ethyl ketone, methyl propyl ketone, methyl butyl ketone, methyl isobutyl ketone and diethyl ketone; ethers such as diethyl ether, ethyl propyl ether, ethyl butyl ether, dipropyl ether, diisopropyl ether, dibutyl ether and dimethoxyethane; nitriles such as acetonitrile, propionitrile, butylonitrile, isobutylonitrile and valeronitrile; substituted or unsubstituted aromatic hydrocarbons such as benzene, toluene, xylene, chlorobenzene and anisole; halogenated hydrocarbons such as dichloromethane (MDC), chloroform, dichloroethane, trichloroethane, dibromoethane, propylene dichloride, carbon tetrachloride and flons; hydrocarbons such as pentane, hexane, heptane and octane; and cycloalkanes such as cyclopentane, cyclohexane, cycloheptane and cyclooctane.

Examples of preferred combinations of the solvents are DMF/THF, DMF/ethyl acetate, DMF/MDC, DMF/toluene, DMF/acetone, DMF/ether, DMF/acetonitrile, THF/acetone, THF/acetonitrile, NMP/toluene, NMP/MDC, NMP/acetonitrile, DMA/toluene, DMA/acetone, DMA/acetonitrile, DMA/MDC, DMF/acetone/acetonitrile and DMF/MDC/acetonitrile.

The solvent used for the present invention can be supplied to the reaction system by appropriately dissolving an amount of the above-mentioned alkali metal carbonate or alkaline earth metal carbonate necessary for the reaction in the solvent.

These solvents may contain water as required. These solvents are used in an amount of about 2 to 200 liters, preferably about 3 to 100 liters, per 1 kg of the compound of the formula (1). The reaction is conducted in the range of -50 to 100 °C, preferably -35 to 40 °C.

The compound of the formula (3) can be obtained as an approximately pure product, by performing, after the reaction is terminated, the usual extraction or crystallization. It is, of course, possible to purify by any other method.

A 3-sulfonyloxy-3-cephem compound shown by the formula (3) can be produced in high purity and a high yield by reacting a 3-hydroxy-3-cephem compound shown by the formula (1) serving as the starting material with a sulfonyl halide in the presence of an alkali metal carbonate or alkaline earth metal carbonate substantially without forming Δ2-cephem compound as a by-product.

A compound shown by the formula (3) produced according to the present invention can be converted to orally administrated antimicrobial agent ceftibuten, for example, by the method described in Pure & Appl. Chem., 59, 1041 (1987). In addition, a compound shown by the formula (3) can be lead to a 3-vinyl-3-cephem compound by the method which is described in Japanese Patent Application No. 247272/1999, which can be converted to orally administrated antimicrobial agent cefixime (Handbook of Latest Antibiotics, 9th ed., Katsuji Sakai, p.83, 1994) by the method which is described in JP-B-20435/1988 *via* deprotection, or to cefdinir (Handbook of Latest Antibiotics, 9th ed., Katsuji Sakai, p.86, 1994).

According to the present invention, substituents R¹, R², and R³ of a compound shown by the formula (1) are not involved in the reaction, i.e., the reaction proceeds independent of the kind, so that a compound shown by the formula (3) can be obtained which can be used as an intermediate for useful non-natural antibacterial agents.

### BEST MODE OF CARRYING OUT THE INVENTION

Although the present invention will be described in detail referring to Examples and Reference Examples below, the present invention is not limited to these Examples at all.

### Example 1

N,N-dimethylformamide (DMF,600 mL) and 52 mL of methanesulfonyl chloride which were cooled at 3 to 5°C were added to a mixture of 180 g of a compound shown by the formula (1) (R¹ = phenylacetamido group; R² = H; R³ = diphenylmethyl group) (1a) and 70 g of sodium carbonate, and the mixture was stirred at 3 to 5°C for 2 hours. The reaction mixture was poured into a mixture of 2L of methylene chloride and 2L of water, and extraction was carried out to give an organic layer, which was twice washed with 2L of water, and the solvents were distilled off at a reduced pressure. To the residue, 2L of 75% methanol was added for crystallization, and the formed crystals were matured at 3 to 5°C for 1 hour, separated by filtration, and dried to give 195 g of the compound shown by the formula (3) (R¹ to R³ are as defined above; R⁴ = methyl group) (3a). Yield, 94% (content of Δ2-cephem compound, 0.2%).
1H NMR(300MHz, DMSO-d₆) δ 3.17(s, 3H), 3.48 (d, J=14Hz, 1H), 3.56(d, J =14Hz, 1H), 3.74(d, J=18Hz, 1H), 4.00(d, J=18Hz, 1H), 5.26(d, J=4.6H z, 1H), 5.82(dd, J=4.6, 8.2Hz, 1H), 6.92(s, 1H), 7.18-7.52(m, 15H), 9.23(d, J=8.2Hz, 1H).

### Reference Example 1

In 1 mL of tetrahydrofuran which was kept at -5°C was dissolved 90 mg of compound (1a). Into the mixture were added 19 *µ* L of methanesulfonyl chloride and 67 *µ*L of triethylamine, and the resultant mixture was stirred at -5°C for 1 hour. The reaction mixture was analyzed by the high-performance liquid chromatography (HPLC). An amount of each product was determined by the external standard method, resulting that the reaction mixture contained 8% of compound (1a) which remained unreacted, 30 mg (26%) of compound 3a, and 71 mg (61%) of the Δ2-cephem compound.

### Reference Example 2

In 1 mL of DMF which was kept at 0 to 5°C was dissolved 100 mg of compound (1a). Into the mixture were added 21 *µ*L of methanesulfonyl chloride and 74 µL of triethylamine, and the obtained mixture was stirred at 0 to 5°C for 1 hour. The obtained reaction mixture was analyzed by HPLC, resulting that the reaction mixture contained 91 mg (82%) of compound (3a) and 11 mg (10%) of the Δ2-cephem compound.

### Reference Example 3

The reaction was carried out in a manner similar to Reference Example 2 except that the temperature of added DMF and that of the reaction were -30°C. HPLC revealed that the reaction mixture contained 98 mg (88%) of compound (3a) and 4 mg (4%) of the Δ2-cephem compound.

### Example 2

DMF (0.4 mL) and 32 *µ*L of methanesulfonyl chloride which were cooled at 3 to 5°C were added to a mixture of 100 mg of the compound shown by the formula (1) (R¹ = phenylacetamido group; R² = H; R³= p-methoxybenzyl group) (1b) and 43 mg of sodium carbonate, and the mixture was stirred at 3 to 5°C for 2 hours. The resultant reaction mixture was poured into a mixture of 20 mL of dichloromethane and 20 mL of water, and extraction was carried out to give an organic layer, which was washed with 20 mL of water, dried over magnesium sulfate, and the solvents were distilled off at a reduced pressure to give a residue, which was purified by the column chromatography to give 122 mg of the compound shown by the formula (3) (R¹ to R³ are as defined above; R⁴ = methyl group) (3b). A Δ2-cephem compound was not found during fractionation by the column chromatography. Yield, 96%.
1H NMR(300MHz, CDCl₃) δ 3.05 (s, 3H), 3.55(d, J=18Hz, 1H), 3.59(d, J=1 6Hz, 1H), 3.66(d, J=16Hz, 1H), 3.77(d, J=18Hz, 1H), 3.79(s, 3H), 4.9 8(d, J=4.9Hz, 1H), 5.16(d, J=12Hz, 1H), 5.22(d, J=12Hz, 1H), 5.83(dd, J=4.9, 9.1Hz, 1H), 6.20(d, J=9.1Hz, 1H), 6.82-7.40(m, 9H).

### Examples 3 to 8

The Reactions were carried out in manners similar to Example 2 except that solvents which are shown below were used to give compound (3b). Results are shown in Table 1. Yields of the Δ2-cephem compound were 0.3% or less.

**Table 1**

| Example | solvent | yield (%) |
|---|---|---|
| 3 | NMP | 92 |
| 4 | DMA | 90 |
| 5 | THF | 85 |
| 6 | MDC/DMF | 85 |
| 7 | ethyl acetate/DMF | 82 |
| 8 | THF/DMF | 85 |

### Examples 9 to 13

The reactions were carried out in manners similar to Example 2 except that alkali metal carbonate or alkaline earth metal carbonate which are shown below were used instead of sodium carbonate to give compound (3b). Results are given in Table 2. Yields of the Δ2-cephem compound were 0.4% or less.

**Table 2**

| Example | alkali metal/alkaline earth metal carbonate | yield(%) |
|---|---|---|
| 9 | potassium carbonate | 94 |
| 10 | lithium carbonate | 92 |
| 11 | calcium carbonate | 84 |
| 12 | strontium carbonate | 82 |
| 13 | barium carbonate | 82 |

### Example 14

Ten mL of DMF and 648 mg of p-toluenesulfonyl chloride which were cooled at 3 to 5°C were added to a mixture of 1 g of compound (1a) and 360 mg of sodium carbonate, and the mixture was stirred at 3 to 5°C for 2 hours. The mixture was treated in a manner similar to Example 2 to give 1.26 g of the compound shown by the formula (3) (R¹ = phenylacetamido group; R² = H; R³ = diphenylmethyl group; R⁴ = p-tolyl group) (3c). A Δ2-cephem compound was not found by fractionation by column chromatography. Yield, 96%.
1H NMR (300MHz, CDCl₃) δ 2.37(s, 3H), 3.42(d, J=18Hz, 1H), 3.58(d, J=16Hz, 1H), 3.65(d, J=16Hz, 1H), 3.77(d, J=18Hz, 1H), 4.99(d, J=4.8 Hz, 1H), 5.81(dd, J=4.8, 8.7Hz, 1H), 6.05(d, J=8.7Hz, 1H), 6.77(s, 1 H), 7.15-7.49(m, 19H).

### Example 15

Ten mL of DMF and 715 mg of p-toluenesulfonyl chloride which were cooled at 3 to 5°C were added to a mixture of 1 g of compound (1b) and 367 mg of sodium carbonate, and the mixture was stirred at 3 to 5°C for 2 hours. The mixture was treated in a manner similar to Example 2 to give 1.27 g of the compound shown by the formula (3) (R¹ = phenylacetamido group; R² = H; R³ = p-methoxybenzyl group; R⁴ = p-tolyl group) (3d). A Δ2-cephem compound was not found by fractionation by column chromatography. Yield, 95%.
1H NMR(300MHz, CDCl₃) δ 2.45(s, 3H), 3.34(d, J=19Hz, 1H), 3.57(d, J=1 6Hz, 1H), 3.62(d, J=19Hz, 1H), 3.64(d, J=16Hz, 1H), 3.80(s, 3H), 4.9 4(d, J=4.8Hz, 1H), 4.98(d, J=12Hz, 1H), 5.03(d, J=12Hz, 1H), 5.79(dd, J=4.8, 9.0Hz, 1H), 6.17(d, J=9.0Hz, 1H), 6.85-7.75(m, 13H).

### Example 16

Three mL of DMF and 0.12 mL of trifluoromethanesulfonyl chloride which were cooled at 3 to 5°C were added to a mixture of 300 mg of compound (1a) and 108 mg of sodium carbonate, and the mixture was stirred at 3 to 5°C for 2 hours. The mixture was treated in a manner similar to Example 2 to give 349 mg of the compound shown by the formula (3) (R¹ = phenylacetamido group; R² = H; R³ = diphenylmethyl group; R⁴ = trifluoromethyl group) (3e). A Δ 2-cephem compound was not found by fractionation by column chromatography. Yield, 92%.
1H NMR(300MHz, DMSO-d₆) δ 3.50(d, J=14Hz, 1H), 3.58(d, J=14Hz, 1H), 3.82(d, J=18Hz, 1H), 4.03(d, J=18Hz, 1H), 5.32(d, J=4.8Hz, 1H), 5.90(dd, J=8.1Hz, 1H), 6.94(s, 1H), 7.20-7.54(m, 15H), 9.26(d, J=8.1Hz, 1H).

### Example 17

Three mL of DMF and 0.13 mL of trifluoromethanesulfonyl chloride which were cooled at 3 to 5°C were added to a mixture of 300 mg of compound (1b) and 119 mg of sodium carbonate, and the mixture was stirred at 3 to 5°C for 2 hours. The mixture was treated in a manner similar to Example 2 to give 348 mg of the compound shown by the formula (3) (R¹ = phenylacetamido group; R² = H; R³ = p-methoxybenzyl group; R⁴ = trifluoromethyl group) (3f). A Δ2-cephem compound was not found by fractionation by column chromatography. Yield, 90%.
1H NMR(300MHz, DMSO-d₆) δ 3.36(d, J=18Hz, 1H), 3.57(d, J=16Hz, 1H), 3.65(d, J=16Hz, 1H), 3.69(d, J=18Hz, 1H), 3.79(s, 3H), 4.98(d, J=4.6Hz, 1H), 5.14(d, J=12Hz, 1H), 5.33(d, J=12Hz, 1H), 5.85(dd, J=4.6, 8.6Hz, 1H), 6.19(d, J=8.6Hz, 1H),6.83-7.40(m,9H).

### Examples 18 to 22

The reactions were carried out in manners similar to Example 2 except that reaction temperatures are changed as shown below to give compound (3b). The results are shown in Table 3. The Δ2-cephem compound was formed at 0.5% or less in each example.

**Table 3**

| Example | temperature (°C) | yield(%) | contents of Δ2-cephem compound (%) |
|---|---|---|---|
| 18 | -30 ~ -25 | 93 | 0.1 |
| 19 | -10 ~ -5 | 92 | 0.1 |
| 20 | 10 ~ 15 | 90 | 0.3 |
| 21 | 25 ~ 30 | 87 | 0.3 |
| 22 | 40 ~ 45 | 72 | 0.5 |

### Example 23

Three mL of DMF and 0.09 mL of methanesulfonyl chloride which were cooled at 3 to 5°C were added to a mixture of 300 mg of compound (1) (R¹ = p-CH30-C6H4-CH=N-; R² = H; R³ = diphenylmethyl group) (1c) and 117 mg of sodium carbonate, and the mixture was stirred at 3 to 5°C for 2 hours. The mixture was treated in a manner similar to Example 2 to give 318 mg of the compound shown by the formula (3) (compound 3g). Yield, 92%. (content of Δ2-cephem compound : 0.2%)
1H NMR (300MHz, CDCl₃) δ 2.72(s, 3H), 2.56(d, J=18Hz, 1H), 3.81(s, 3H), 3.85(d, J=18Hz, 1H), 5.16(d, J=4.8Hz, 1H), 5.34(d, J=4.8Hz, 1H), 6.88-7.76(m, 14H), 6.98(s, 1H), 8.46(s, 1H).

The developing solvent of the column chromatography used in Examples 2 to 22 was benzene : hexane = 4:1 to 6:1.

### Reference Example 4

### [Synthesis of ceftibuten from compound (3a)]

Compound (3a) obtained in Example 1 can be converted to ceftibuten by the method described in Pure & Appl. Chem., 59, 1041 (1987).

Compound (3a) is reacted with phosphorus pentachloride/pyridine reagent using methylene chloride as a solvent, and the reaction mixture is cooled at -35°C, and treated with methanol to give 7-amino-3-methanesulfonyloxycephem hydrochloride (4), which is reduced by the action of zinc in acetic acid to give 7-amino-3-cephem hydrochloride (5), which is reacted with an acid chloride (6) in the presence of a base to give a precursor (7), which is deprotected (final deprotection) to give ceftibuten. 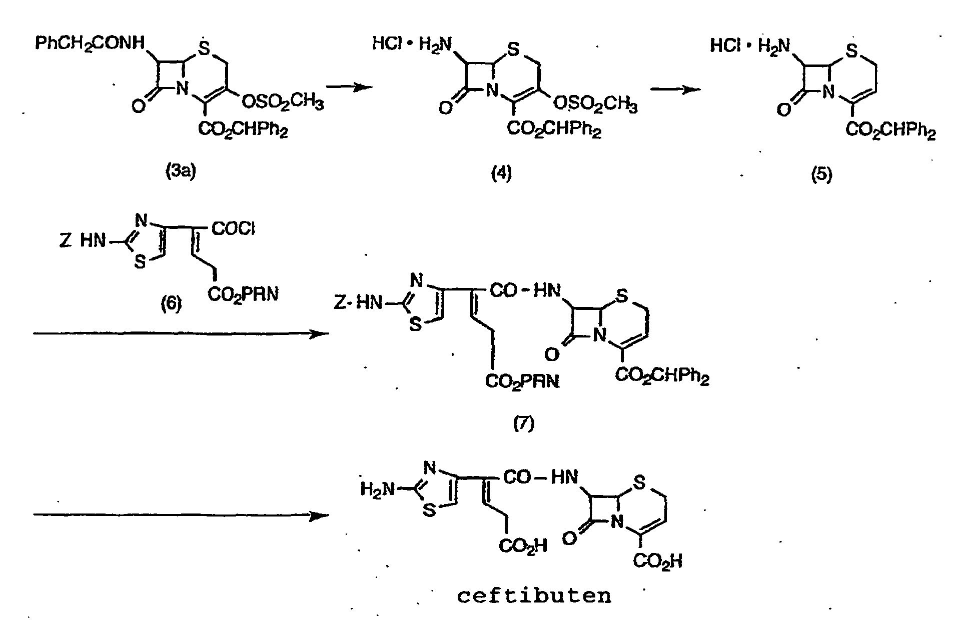 [Ph = phenyl, Z = benzoyl, PRN = -CH₂CH = C(CH₃)₂]

### Reference Example 5

### [Synthesis of cefixime from compound (3a)]

Compound (3a) obtained in Example 1 is converted by the method described in Japanese Patent Application No. 247272/1999 to 3-vinyl-3-cephem compound (8), which is reacted with the phosphorus pentachloride/pyridine reagent using methylene chloride as a solvent, and the reaction mixture is cooled at -35°C, and treated with methanol to give 7-amino-3-vinyl-3-cephem hydrochloride (9), to which phenol is added, and the obtained mixture is heated at 45°C for 1 hour for reaction to give 7-amino-3-vinyl-3-cephem-4-carboxylic acid (10), which is subjected to the reaction with respect to the side chain at position 7, and the final deprotection, by the method described in JP-B-20435/1988 to give cefixime.

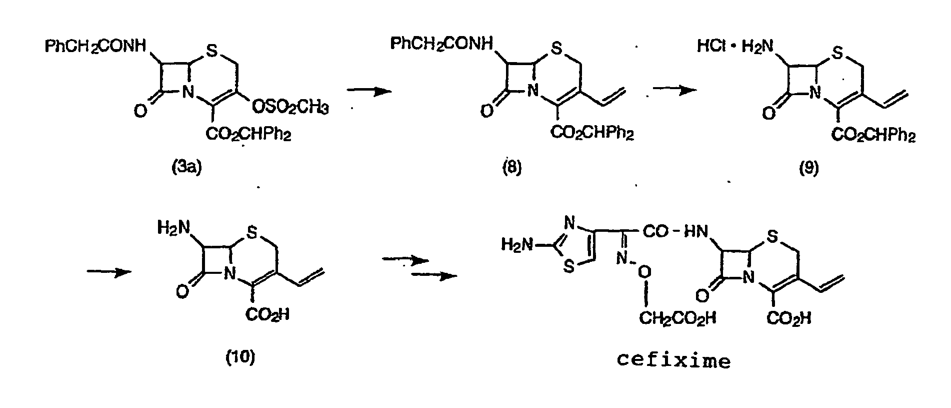

### INDUSTRIAL APPLICABILITY

The present invention can produce a 3-sulfonyloxy-3-cephem compound at high purity and a high yield which is an intermediate for useful non-natural antibacterial agents which have wide antibacterial spectra substantially without forming a Δ2-cephem compound as a by-product.

## Claims

1. A method for producing a 3-sulfonyloxy-3-cephem compound shown by the formula (3) comprising reacting a 3-hydroxy-3-cephem compound shown by the formula (1) with a sulfonyl halide shown by the formula (2) in an organic solvent in the presence of an alkali metal carbonate or alkaline earth metal carbonate. 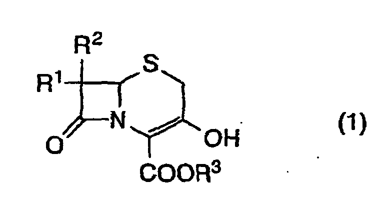 [R¹ is hydrogen atom, halogen atom, protected amino group or ArCH=N- (Ar is a substituted or unsubstituted aryl group); R² is hydrogen atom, halogen atom, C₁-C₄-alkoxy group, C₁-C₄-acyl group, protected hydroxy group or C₁-C₄-alkyl which may have protected hydroxyl group as a substituent; R³ is hydrogen atom or carboxylic acid protective group]
**R**^{**4**}**SO**_{**2**}**X** **(2)**
(R⁴ is C₁-C₄-alkyl which may have a substituent or aryl which may have a substituent; X is halogen atom) 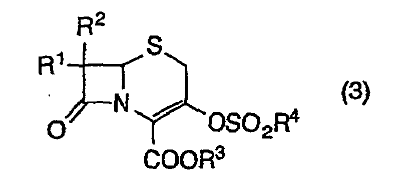 (R¹ to R⁴ are same as above).

2. A process as defined in claim 1 wherein the organic solvent is at least one selected from the group consisting of an amide solvent and cyclic ether solvent.

3. A process as defined in claim 1 wherein the sulfonyl halide compound (2) is methanesulfonyl chloride, trifluoromethanesulfonyl chloride or p-toluenesulfonyl chloride.

4. A process as defined in claim 1 wherein the alkali metal carbonate or alkaline earth metal carbonate is sodium carbonate, potassium carbonate, lithium carbonate, magnesium carbonate, calcium carbonate, barium carbonate or strontium carbonate.

## Patentansprüche

1. Verfahren zur Herstellung einer durch die Formel (3) gezeigten 3-Sulfonyloxy-3-cephem-Verbindung umfassend die Umsetzung einer durch die Formel (1) gezeigten 3-Hydroxy-3-cephem-Verbindung mit einem durch die Formel (2) gezeigten Sulfonylhalogenid in einem organischen Lösungsmittel in Anwesenheit eines Alkalimetallcarbonats oder Erdalkalimetallcarbonats 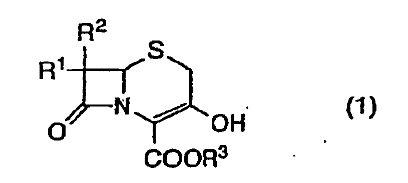 [R¹ ist ein Wasserstoffatom, Halogenatom, eine geschützte Aminogruppe oder Ar-CH=N- (Ar ist eine substituierte oder unsubstituierte Arylgruppe); R² ist ein Wasserstoffatom, ein Halogenatom, eine C₁-C₄-Alkoxygruppe, C₁-C₄-Acylgruppe, geschützte Hydroxygruppe oder C₁-C₄-Alkyl, das eine geschützte Hydroxylgruppe als Substituenten aufweisen kann; R³ ist ein Wasserstoffatom oder eine Carbonsäure-Schutzgruppe]
R⁴SO₂X (2)
(R⁴ ist C₁-C₄-Alkyl, das einen Substituenten aufweisen kann, oder Aryl, das einen Substituenten aufweisen kann; X ist ein Halogenatom) 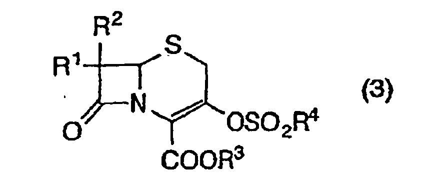 (R¹ bis R⁴ sind dieselben wie vorstehend).

2. Verfahren wie in Anspruch 1 definiert, in dem das organische Lösungsmittel mindestens eines ausgewählt aus der Gruppe bestehend aus einem Amidlösungsmittel und einem cyclischen Etherlösungsmittel ist.

3. Verfahren wie in Anspruch 1 definiert, in dem die Sulfonylhalogenidverbindung (2) Methansulfonylchlorid, Trifluormethansulfonylchlorid oder p-Toluolsulfonylchlorid ist.

4. Verfahren wie in Anspruch 1 definiert, in dem das Alkalimetallcarbonat oder Erdalkalimetallcarbonat Natriumcarbonat, Kaliumcarbonat, Lithiumcarbonat, Magnesiumcarbonat, Calciumcarbonat, Bariumcarbonat oder Strontiumcarbonat ist.

## Revendications

1. Procédé de production d'un composé 3-sulfonyloxy-3-cephem représenté par le formule (3), comprenant la réaction d'un composé 3 -hydroxy-3-cephem représenté par la formule (1), avec un halogénure de sulfonyle représenté par la formule (2), dans un solvant organique, en présence d'un carbonate de métal alcalin ou d'un carbonate de métal alcalinoterreux, 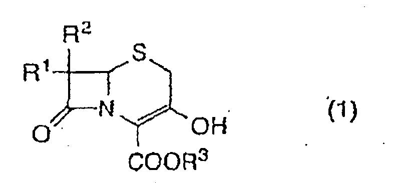 [R¹ représente un atome d'hydrogène, un atome d'halogène, un groupe amino ou Ar-CH=N- protégé (Ar est un groupe aryle substitué ou non substitué) ; R² représente un atome d'hydrogène, un atome d'halogène, un groupe alcoxy C₁-C₄, un groupe acyle C₁-C₄, un groupe hydroxy protégé ou un groupe alkyle C₁-C₄, qui peut avoir un groupe hydroxyle protégé comme substituant ; R³ représente un atome d'hydrogène ou un groupe acide carboxylique protecteur].
R⁴SO₂X (2)
[R⁴ représente un groupe alkyle C₁-C₄ qui peut avoir un substituant, ou un groupe aryle qui peut avoir un substituant ; X représente un atome d'halogène] 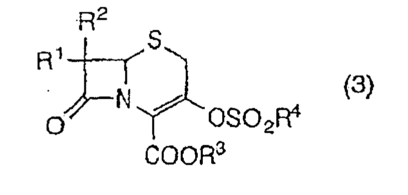 (R¹ à R⁴ sont les mêmes que ci-dessus).

2. Procédé selon la revendication 1, dans lequel le solvant organique est au moins l'un choisi au sein du groupe consistant en un solvant amide et en un solvant éther cyclique.

3. Procédé selon la revendication 1, dans lequel le composé halogénure de sulfonyle (2) est le chlorure de méthanesulfonyle, le chlorure de trifluorométhanesulfonyle ou le chlorure de p-toluène-sulfonyle.

4. Procédé selon la revendication 1, dans lequel le carbonate de métal alcalin ou le carbonate de métal alcalinoterreux est le carbonate de sodium, le carbonate de potassium, le carbonate de lithium, le carbonate de magnésium, le carbonate de calcium, le carbonate de baryum ou le carbonate de strontium.
